# EUROPEAN PATENT SPECIFICATION

(11) **EP 3 906 956 B1**
(45) Date of publication and mention of the grant of the patent: **25.10.2023**
(21) Application number: 21162424.2
(22) Date of filing: 14.11.2013
(51) Int. Cl.: A61M 16/06

(54) **A PATIENT INTERFACE**
PATIENTENSCHNITTSTELLE
INTERFACE PATIENT

(43) Date of publication of application: 10.11.2021
(62) Divisional of application: 13192948.1
(73) Proprietor: ResMed Pty Ltd, Bella Vista, NSW 2153 (AU)
(72) Inventor: NICKOL, Johannes, 80636 Munich (DE); EIBL, Robert, 83646 Bad Tölz (DE); BURZ, Sebastian, 87656 Germaringen (DE); BIENER, Achim, 85445 Aufkirchen (DE); LANG, Bernd, 82166 Gräfelfing (DE); NIBU, Adel, 82284 Grafrath (DE); KIRCHBERGER, Andreas, 83714 Miesbach (DE); ROTHFUSS, Jens, 80807 Munich (DE)
(74) Representative: Vossius & Partner Patentanwälte Rechtsanwälte mbB

(56) References cited:
- WO-A1-2004/041342
- WO-A1-2007/068044
- WO-A1-2009/062265
- WO-A1-2013/050911
- WO-A1-2013/136221
- WO-A2-2012/131001
- US-A- 5 770 220

## Description

### TECHNICAL FIELD

The present technology relates to a patient interface; particularly used for treatment, e.g., of sleep disordered breathing (SDB) such as obstructive sleep apnea (OSA), and/or for respiratory care, e.g., of chronic obstructive pulmonary disease (COPD).

The invention relates to a patient interface.

### BACKGROUND

Patients who suffer from obstructive sleep apnea can use continuous positive airway pressure (CPAP) therapy, to maintain the upper airway open while they are asleep. CPAP therapy is applied to the patient using a mask, tubing, and a flow generator. All of these components encompass the air delivery system provided to the patient.

One problem for patients who undergo CPAP therapy is irritation and skin ailments, e.g., sores, caused by wearing a mask. The problem is a result of the mask cushion compressing or applying pressure to the patient's skin in a similar region for a prolonged length of time. Blood capillaries may be squashed or impinged upon which may cause a blockage or partial blockage to circulation. This may result in inflammation and/or redness of the skin and/or facial numbness. These problems may cause a patient to cease therapy or to change interface settings, such as position of the interface or pressure with which it is held on the face which may negatively influence therapy.

A known solution includes use of two or more different masks with differing face contacting properties and wearing them alternately. Another known solution includes switching between two different styles of mask, e.g., switch between a full-face mask and a nasal mask or switch between a nasal cushion and nasal pillows. Yet another known solution includes repositioning the mask on the patient's face if the patient wakes up during the night.

These approaches, however, have several drawbacks, e.g., require the purchase of multiple masks and/or prevent patient from a full night of sleep. Another problem may be sweat or humidification caused by wearing a mask. The humidity or liquid can block the patient's skin ducts, which may cause irritation.

Another problem patients have with masks is that they may cause discomfort, and it can be difficult for patients to relax when wearing one. If a patient cannot relax enough to fall asleep, then the mask is ineffective as treatment for sleeping disorders as it is preventing the patient from having a full nights sleep.

Another problem with masks may be mask leak. Leaks between the cushion and the patient's skin can be generated after the patient has fallen asleep due to the patient's movements. These leaks may cause the mask to stop delivering appropriate treatment by reducing the mask pressure and/or by waking the patient. Either of these occurrences may result in the patient not receiving a good night's sleep. Leaks often tend to occur at specific regions of the cushion seal. It is known that additional pressure to stop the leaks may be applied by tightening headgear straps. However, this approach tends to tighten the entire cushion against the patient's face, which can lead to discomfort or sores, as referred to above.

WO 2007/068044 describes a patient interface such as a mask that may be used for treatment of sleep disordered breathing (SDB) such as obstructive sleep apnea (OSA). A cushion of the mask may include fluid-filled bladders that may be pressurized with a fluid, which includes gases, e.g., air, and liquids. The pressurized fluid may be provided to the bladders with a pulsing effect, and the fluid contained in the bladders may be heated in the bladders, or may be heated before being communicated to the bladders.

Thus, there is a need for an improved patient interface that does not suffer from the drawbacks of the prior art, and which may provide additional advantages as will be apparent from the disclosure below.

WO 2013/136221 A1 relates to a cushion member for a patient interface device that includes a main body portion including a plurality of chambers and a self-sealing cap portion coupled to the main body portion as well as to an apparatus and a method for selectively adjusting the internal pressure of the chambers of such a cushion.

WO 2013/050911 A1 relates to a cushion for use with a mask in delivering a flow of breathing gas to a user including a first portion adapted to contact a surface of a user, a second portion adapted to be coupled to a mask shell, and a wall portion extending between the first portion and the second portion, the wall portion including a chamber formed therein, the chamber having one of an electro-rheological fluid or a magneto-rheological fluid disposed therein. Furthermore, it is said that the cushion includes a means for selectively varying the apparent viscosity of the fluid disposed within the chamber.

WO 2004/041342 A1 relates to a comfortable low-leak mask assembly for use with Non-Invasive Positive Pressure Ventilation (NIPPV), which may include headgear having straps that are substantially inextensible and/or micro-adjustable; and/or a mask and/or cushion that includes various structures to allow enhanced/tailored sealing and/or fit at selected locations on the patient's face.

WO 2012/131001 A2 relates to a patient interface comprising a member for contacting the skin of a patient during use, wherein at least a part of said member comprises a substance, wherein the substance is released to the patient's skin during use.

WO 2009/062265 A1 relates to a cushion assembly for use with a respiratory mask includes a bladder filled with the combination of a gel having a first indentation hardness and a gel having a second indentation hardness.

### SUMMARY

The invention is set out in the appended set of claims.

The following forms and aspects thereof are described and illustrated in conjunction with systems, tools and methods which are meant to be exemplary and illustrative, not limiting in scope.

The present technology is expressed by the features of the independent claims and aspects. The dependent claims and aspects refer to preferred forms.

The present technology relates to a patient interface for providing breathable gas to a patient where at least one property of the interface or of a substance, such as a fluid, included in the interface may be changed or incited to change. The property may be optionally changed by means in or on the interface or, in addition or alternatively, by means communicating with the interface such as by wire-communication, wireless-communication or hydraulic and/or fluid communication. Properties in particular at or adjacent areas of the interface that engage the face of the patient may be changed, and preferably the properties are of fluids or liquids that are contained in the interface. A preferred property is temperature and/or pressure. Additional or alternative properties will become apparent from the below discussion. Also, the change in properties may be used for conveying signals to the patient's tissue e.g. to stimulate blood circulation.

The above forms and aspects may provide the following advantages, effects and/or benefits.
a) Skin tissue may be stimulated by temperature changes in the mask cushion, thus blood circulation may be improved, leading to less/reduced pressure sores when wearing the mask for an extended time period.
b) Changes in cushion cavity internal pressure may affect the shape of the cushion, thus pressure points on the patient's face may shift slightly, possibly giving the skin time to recover.
c) Changes in cushion cavity internal pressure may cause a kind of "massage" effect which stimulates blood circulation.
d) Cushion cavity may be filled with a paste-like skin care substance, and walls enclosing the cavity may be at least partly permeable to that substance, so that a pressure rise may be used to release a small amount of the paste-like substance onto the patient's face.
e) Heated fluid supplied to the mask cushion may also be used to heat the wall of the breathing tube, and thus prevent condensation in the tubing, thus possibly eliminating the need for an electrical heating.

Changes in pressure described above may preferably require only one supply line/tube per cushion cavity and may work in three different ways:
a) Cushion cavity internal pressure may rise with increasing therapy pressure and decrease with decreasing pressure. This may stiffen the mask cushion during the inspiratory phase, and may relax the cushion during the expiratory phase. This may cause a "massage" effect in frontal-dorsal direction on the face.
b) Cushion cavity internal pressure may decrease with increasing therapy pressure and may increase with decreasing pressure. This may relax the mask cushion during the inspiratory phase, allowing the cushion to slightly bulge outward, and stiffen the cushion during the expiratory phase. This causes a "massage" effect in distal-central direction on the face
c) The pressure in the cushion cavity may changes independent of the therapy pressure swings, e.g. in a defined interval that can be set by the user, so that the pressure points on the patient's face shift during therapy in a defined interval (e.g. 1 hour higher pressure, 1 hour lower pressure)

The changes in temperature described may be implemented by replacing the fluid contained in the cushion cavity by a fluid of higher or lower temperature, thus changing the cushion temperature. This may require two supply lines per cavity, so that a circulation can be established. Because of higher thermal capacity of liquid, a liquid may in some cases be preferable over a gas for inciting change to temperature.

The release of paste-like substance can be effected either by pumping gas into the cushion cavity, and/or by pumping additional paste-like substance. In some forms the pumping may not be continuous, but possibly in pulses every e.g. 1 hour or so - to give the skin time to absorb the released skin care substances.

The warm fluid supplied to the mask may optionally be used to heat the tubing supplying the patient's breathing air by e.g. coiling the fluid supply tubing around the air supply tubing.

The pressure change and supply of liquid may be effected by industry standard components: a small pump such as a piezoelectric pump, heating/cooling elements such as a Peltier element or cooling fan, temperature and/or pressure sensors as needed. Possibly all (or at least all) of the peripheral equipment required to cause and to control e.g. the pressure/temperature changes may be contained in a separate device, to keep the mask itself lightweight as possible. In addition to the exemplary aspects, forms and advantages described above, further aspects, forms and advantages will become apparent by reference to the figures and by study of the following detailed descriptions.

### BRIEF DESCRIPTION OF THE FIGURES

Exemplary forms are illustrated in referenced figures. It is intended that the forms and figures disclosed herein are to be considered illustrative, rather than restrictive. The disclosure, however, both as to organization and method of operation, together with objects, features, and advantages thereof, may best be understood by reference to the following detailed description when read with the accompanying figures, in which:
**Fig. 1** shows a side view of a patient interface in accordance with one form of the present technology;
**Figs. 2** shows a perspective view onto the patient contacting side of an exemplary form of a cushion of the patient interface in accordance with the present technology;
**Figs. 3** shows a perspective view onto the interface contacting side of an exemplary form of a cushion of the patient interface in accordance with the present technology;
**Figs. 4** shows a perspective view onto the patient contacting side of an exemplary form of a cushion of the patient interface in accordance with the present technology;
**Figs. 5** shows a side view onto the interface contacting side of an exemplary form of a cushion of the patient interface in accordance with the present technology;
**Figs. 6** shows a cross sectional view onto the interface along lines VI-VI in Fig. 6;
**Fig. 7** shows a perspective transparent view of an exemplary form of a cushion of the patient interface in accordance with the present technology;
**Fig. 7A** is a section of Fig. 7 showing a possible internal construction of the cushion in Fig. 7;
**Fig. 7B and 7C** show exemplary diagrams of embodiments of systems in accordance with the present technology; and
**Figs. 8 to 11** show side views of a patient interface in accordance with various exemplary forms of the present technology.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated within the figures to indicate like elements.

### DETAILED DESCRIPTION

The various exemplary forms of the present technology are directed to a patient interface such as a mask that is operable in different modes of operation. The patient interface may be useful for treatment, e.g., of sleep disordered breathing (SDB) such as obstructive sleep apnea (OSA), and/or for respiratory care for patients wearing the interface for longer than just during night time, such as, e.g., patience suffering from chronic obstructive pulmonary disease (COPD), where the mask is intended particularly to mitigate issues such as pressure sores arising from long term exposure to treatment.

The patient interface has a cushion for contacting a face of a patient and the different modes of operation of the patient interface may include changes that are made to properties of fluid contained in the cushion.

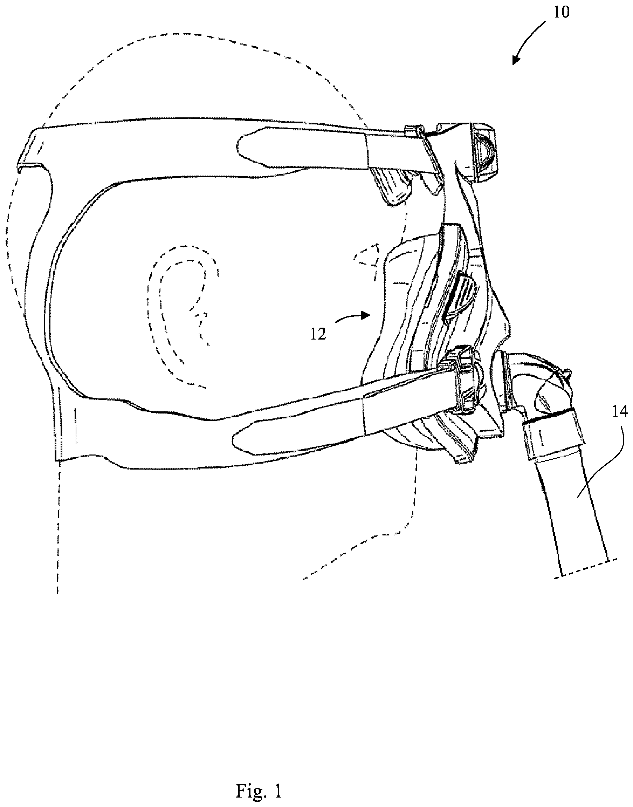

In Figs. 1 and 8 to 11 various forms of a patient interface 10 in accordance with the present technology are seen, all optionally strapped to a head of a patient. Such patient interface may be positioned to contact the face of the patient and surround his mouth and/or nose. The patient interface is preferably a breathing mask 10 including frame components and a cushion 12 for contacting and sealing the mask against the patient's skin. In use, breathable gas is provided to interface 10 at elevated pressure via a breathing hose 14 and then to the patient via patient interface 10. Here, the cushion 12 also seals the mask interior against the environment by sealingly contacting the patient's skin, particularly by limiting leakage of the breathable gas to the ambient environment, particularly at the area of contact of the interface 10 with the patient. The cushion is preferably a fluid filled cushion, e.g., filled with a gas such as air, a gel such as silicone gel, or with liquid, such as water.

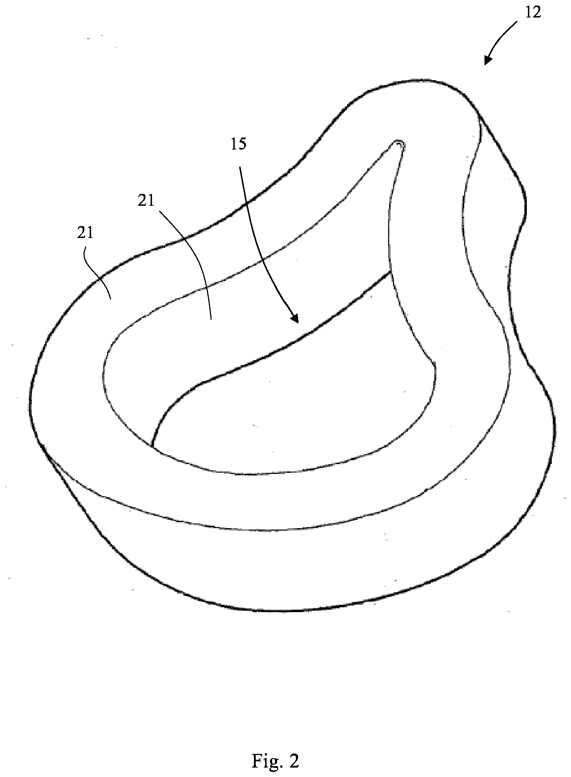

With attention drawn to Fig. 2 one form of the cushion 12 can be seen having an opening 15 that is formed therethrough or defined thereby. The opening is adapted to form a mask interior and to receive a wearer's nose and/or mouth. Via the opening, breathable gas can flow towards or away from the patient. Here, cushion 12 and opening 15 are illustrated having generally triangular shapes, however other shapes are also applicable for either the cushion 12 or opening 15 in accordance with the present technology. Preferably, cushion 12 has outer walls 21 that enclose an interior volume or cavity (not shown in Fig. 2) where the fluids may be contained. The walls of the cavity may vary in thickness along the circumference of the cushion.

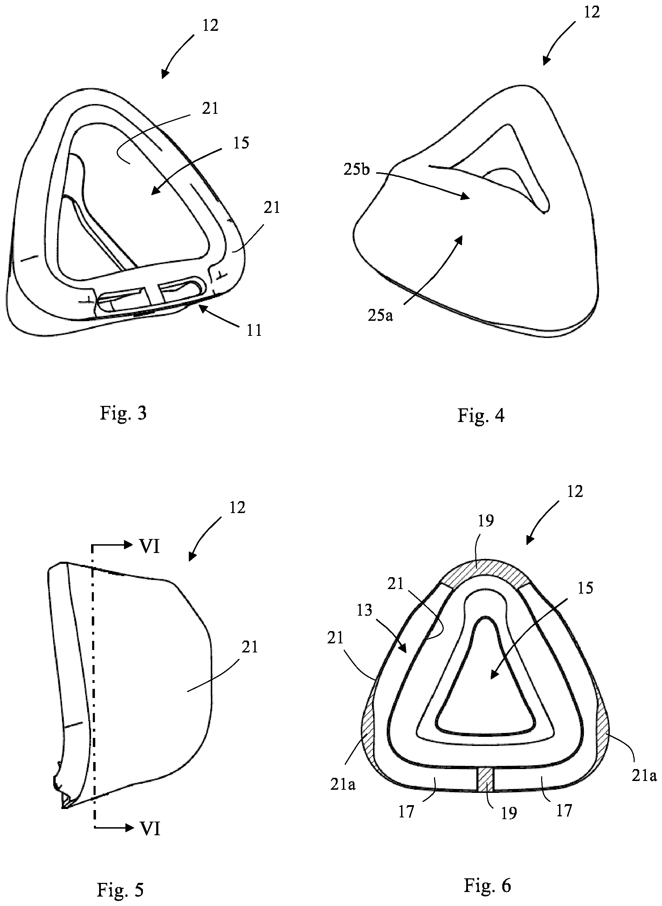

Attention is drawn to Figs. 3 to 6 showing another form of a cushion 12 in accordance with the present technology. Fig. 3 is a perspective view of the cushion taken from the side of the cushion that attaches to interface or mask 10, such as to the frame components and/or a shell of the mask. Fig. 4 is a perspective view of the cushion taken from the side that contacts the face of the patient. Fig. 5 is a side view of the cushion and Fig. 6 is a cross sectional view of the cushion taken in a plane indicated by the roman characters VI-VI in Fig. 6.

The cushion 12 also provides an opening 15 via which breathable gas can flow towards and/or away from the patient, outer walls 21 and at least one interior volume or cavity 13 (see, e.g., Fig. 6) that is enclosed by the outer wall. The cushion can also be seen in, e.g. Fig. 3, to include at least one, preferably two apertures 11 that extends through the outer wall for allowing, e.g., fluid, communication between the at least one interior volume 13 and the ambient environment outside of the cushion. Typically a pump and/or seal (both not shown) may be located at or adjacent the apertures to avoid leakage of pressure and fluid from the interior volume/cavity to the ambient environment. In Fig. 3, two such apertures 11 are shown, one possibly leading fluid into and the other possibly leading fluid out of the interior volume. Alternatively, the cushion may comprise two cavities 13 or compartments 17 (see below discussion) while each aperture is in communication with one of them. Optionally, the portion of the outer walls 21 in which the aperture/apertures is/are formed is at a portion that resides on a side of the cushion that contacts or is adjacent to the mask and/or the frame components of the mask. It is noted that in forms of the present invention where temperature change are made two openings per cavity may be required, so that fluid can preferably circulate. In forms of the present invention where only pressure change are made only one opening may be required.

Further, as can be seen from, e.g., Fig. 6, the interior volume of cavity 13 of the cushion in one form of the present technology may be divided by barriers 19 into two or more compartments 17 (here two) that may be sealed from each other or be in fluid communication one with the other via e.g. a passage (not shown in Fig. 6) that is formed e.g. through one of the barriers. Each compartment 17 may also be associated with an aperture 11, so that e.g. each aperture 11 seen in Fig 3 may in this form of the cushion function to both lead fluid into and out of the respective compartment.

Referring to Fig. 6 it can be seen that the outer walls 21 of the cushion may include thickened portions 21a preferably at or adjacent at least some of the corners or vertices of the cushion; for example in order to assist maintaining the shape and/or functionality of the cushion during use. Here, as can be seen in Fig. 6, two thickened portion 21a may be located each at a respective lower corner of a triangular shaped cushion, while optionally at the upper corner or apex of the cushion one of the barriers 19 is located, to accordingly provide to the cushion a stiffened structure that assists maintaining the shape and/or functionality (such as cushioning properties) of the cushion during use.

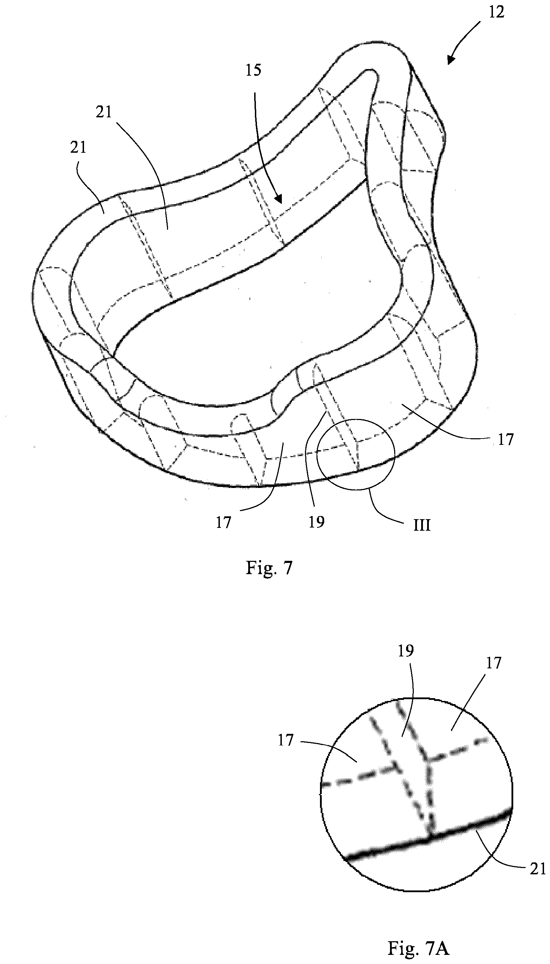

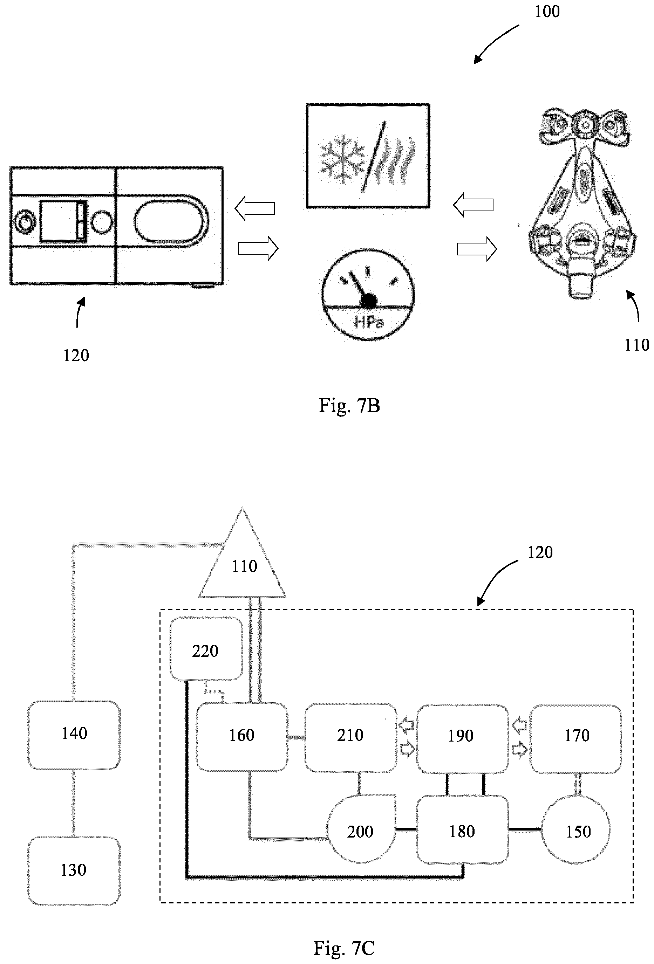

With attention further drawn to Fig. 7 one form of an interior volume 13 can be seen formed of a plurality of compartments 17 with barriers 19 that are placed or extend between adjacent compartments 17. As further seen in Fig. 7A, the barriers 19 may be formed to seal communication between adjacent compartments 17 by, e.g., fully extending between the outer walls 21 of cushion 12. Here the individual compartments sealed one from the other can be individually supplied with fluid while, as discussed, one or more properties of the fluid may be varied for one or more of the individual compartments.

In accordance with the various forms of the present technology, changes to properties of the interface or cushion, particularly of the fluid within the cushion may be affected. This may be implemented by installing a means such as, e.g., a micro pump (e.g. a piezoelectric micro pump), and/or some heating and/or cooling means, in functional relationship with and/or in the patient interface including also optionally in the cushion, or more preferably outside of the interface such as in a device that is either separate or integral with a flow generator supplying (in addition to breathable gas) also fluid to the cushion Such means may be pre-set to alter or incite change to pressure and/or temperature properties of, e.g., the fluid in cushion 12. Since here the operation of the means for inciting change in interface 10 is pre-determined there may be no need to provide any dedicated control means for controlling its operation, and Fig.1 for example may represent such a form of interface 10.

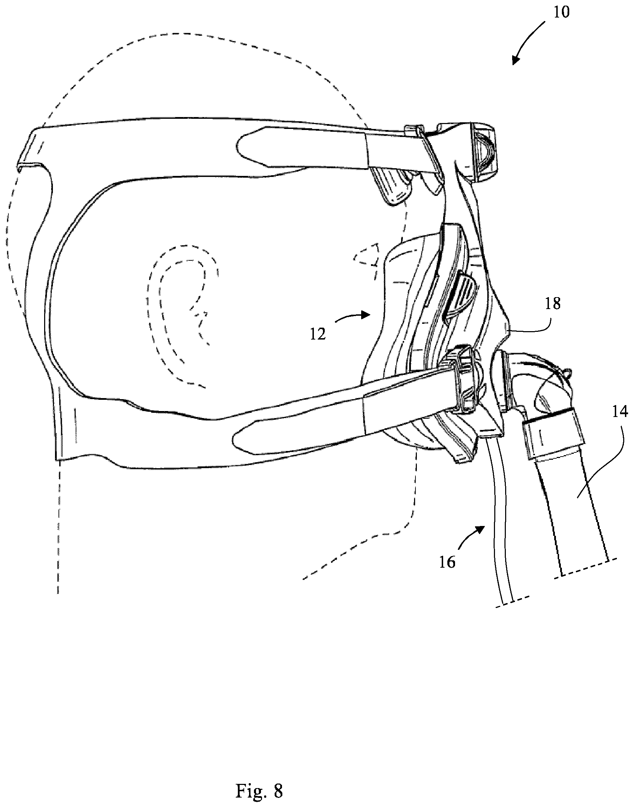

Changes of temperature in supplied liquid fluid may be induced by heating the fluid with e.g. a Peltier element and/or e.g. with means integrated into a humidifier of the flow generator that possibly work together with the humidifier's heating plate Cooling of such heated fluid can be implemented e.g. with a fan and/or heat sink. Fig. 7B schematically shows an exemplary diagram of an embodiment of a system 100 that includes a control means 120 in functional relationship with a patient interface 110 for inciting change to pressure and/or temperature of fluid in a cushion of the patient interface. Fig. 7C schematically shows an exemplary diagram of a patient interface 110 and an embodiment of a control means 120 in functional relationship with the interface, and a flow generator 130 for supplying breathable gas to the patient interface via a ventilation hose 140 (such as e.g. hose 14 seen in Figs. 1 and in Figs. 8-11 discussed below). Control means 120 in this embodiment includes a fan 150, a cooling body 170, a controlling electric circuit 180, a heating/cooling element 190 (such as a Peltier element), a pump 200, a temperature transfer body 210, command lines 160 for communicating medium e.g. air/liquid (such as command lines 16, 161, 162 seen in Figs. 8-11 discussed below) and a sensor 220 such as a temperature sensor.

According to the diagram seen in Fig. 7C, sensor 220 may measure temperature of the medium supplied to the patient interface (e.g. mask) in a location at the command lines (e.g. supply tube/s), in proximity to the flow generator (e.g. medium supply unit).

In some embodiments it may also be possible to measure the temperature at the patient interface (e.g. mask) itself. The measured value may then be communicated to electric circuit 180 (i.e. control electronics) which may then compare the actual value to a set value. The set value may either be pre-set (constant), user-defined (selected) or software defined (for temperature cycling).

Based on the difference between actual value and set value the control electronics may then activate either the heating/cooling element 190 to raise or lower the temperature. The control electronics may additionally be equipped with an ambient temperature sensor of its own, so as to determine the actual temperature at the mask by calculating the temperature loss resulting from ambient temperature as the medium travels through the supply tube. To achieve a similar effect, it may alternatively be desirable for sensor 220 to also measure the temperature of medium returning from the mask, and from the discrepancy between these values the control electronics may then derive the temperature loss due to ambient temperature.

The upper temperature to which the fluid is heated may be about 37°C, the lower temperature to which it is cooled may be about 20°C. However other temperature thresholds and/or a larger delta T (upper termperature - lower temperature) may also be preferred. Furthermore, possibly a user and/or a physician may adjust the temperature limits in a certain range by programming the device used for heating/cooling the fluid. Changes to pressure of a gaseous fluid in the cushion, desirably may be created by forming pressure swings in a fixed interval, e.g. cycling between about 4hPa and 30hPa or preferable between about 10hPa and about 20hPa above ambient, or by causing the pressure supplying device to maintain a pressure of e.g. 10hPa above the supplied therapy pressure to maintain a proper seal. This may be of particular advantage in case of bi-level therapy where the flow generator switches between an inspiratory and an expiratory pressure level. Alternatively or in addition, such means may include a control means and at least one sensor to control its operation based on one or more sensed parameters. Such sensor and/or sensed parameters may include pressure, temperature and/or flow parameters/sensors. Fig. 1 for example may represent such a form of interface 10. In accordance with some forms of the present technology, increasing, e.g., pressure in the cushion and in particular of the fluid in the cushion may assist to control and adjust the spring characteristics of the cushion to the patient's face. Also, it may assist to improve seating and comfort of the interface upon the patient's face, and improve synchronization with therapy settings. Also, pressure swings between the pressure of the breathable gas and the internal pressure of the cushion may counteract each other. For example, a high therapy pressure during inspiration may correspond with a low internal pressure of the cushion and vice versa. That way, it may be possible for the pressureless cushion to slightly bulge outward under inspiration pressure, applying the sealing force more to the outward area of the cushion membrane (see, e.g., indicated 25a in Fig. 4) which then forms the portion of the cushion substantially contacting and sealing against the face of the patient. During the lower expiration pressure the now pressurized cushion may stiffen itself, thus applying the sealing force more to the inward area of the cushion (see, e.g., indicated 25b in Fig. 4) which in turn then forms the portion of the cushion substantially contacting and sealing against the face of the patient. This may create a kind of "massage" effect on the patient's face possibly in a lateral-central direction of the patients face, thus stimulating blood circulation. In addition, reddening or pressure marks may be avoided by having changing portions of the patient skin which are subject to interface contact. Increasing pressure in a cushion including liquids or being provided with liquids that comprise skin-care substances for treating the skin may cause such skin-care substances to be released towards the skin of the patient though, e.g., minute apertures or pores formed in the outer walls 21 of the cushion.

In some forms of the present technology, cavity internal pressure may alternatively increase with increasing therapy pressure and decrease with decreasing therapy pressure. This may urge the cushion to stiffen itself, and vice versa, in accordance with therapeutic pressure increase/decrease so that the cushion relaxes with low therapy pressure. This may also improve the seal between the cushion and the face of the patient and thus require less headgear tension. Also this may create a pumping/massaging motion in a frontal-dorsal direction instead of lateral-central direction as described already above.

In some forms of the present technology, slow pressure cycles in the cavity of a cushion may decouple pressure changes in the cavity from therapy pressure swings. And in yet other forms pressure cycles may not be present at all or at least for some periods of time.

In accordance with one form of the present technology, the following three therapeutic procedures (as mentioned above) are preferably applied separately or cumulatively: 1. A therapeutic procedure characterized by temperature change between warm and cool over several minutes which may stimulate the tissue, 2. A therapeutic procedure characterized by pressure swings which serve to vary the areas of high force on the patient's face, and which also may stimulate the tissue by a kind of "massage" effect, and/or 3. A therapeutic procedure characterized by release of skin-care substances, where defined amounts of (e.g. oily/pasteous/high viscous) substance may be released from the cushion e.g. constantly or intermittently, such as every half-hour/hour by a quick "pressure impulse" (i.e. preferably not a continuous emission). In accordance with the preferable separation between the above mentioned therapeutic procedures, each procedure may be implemented preferably without the other procedures being present, or in other words, preferably therapeutic procedure 1, 2 or 3 may be applied either separately or in combination.

Attention is now drawn to Figs. 8 to 11. In accordance with some forms of the present technology, a command means in the optional form of a command line 16, communicating signals either directly or indirectly with interface 10, here cushion 12, may affect change to properties of the interface or cushion, particularly of the fluid within the cushion. In these figures, command line 16 is illustrated communicating optionally indirectly with cushion 12 via e.g. a frame 18 of the patient interface, however direct communication of command line 16 with cushion 12 is equally applicable in accordance with some forms of the present technology. Command line 16 may, at its end connected to interface 10, be connected to or in communication with a sensor, such as a pressure sensor, a flow sensor, a temperature sensor, (etc.) The other end of command line 16 may, directly or indirectly, be connected to a control unit (not shown).

Possibly the sensor(s) may be located in some forms of the present technology on the mask body itself (e.g. frame 18), thus not directly in the cushion. The sensor(s) used may be industry standard pressure and/or temperature sensor(s) which communicate its/their signals electrically to the control unit. However, to minimize the amount of communication lines, it may be possible to measure the fluid temperature only in the control unit and interpolate the temperature present at the patient's face through industry standard or other software algorithms, and/or to measure the pressure only at the control device, and interpolate the pressure through the software algorithms. In such forms, preferably only the cushion pressure supply line remains as a connection to the patient as e.g. indicated by the single line 16 in Fig. 8 (in addition to the breathing hose) in case of gaseous fluid being supplied towards the cushion.

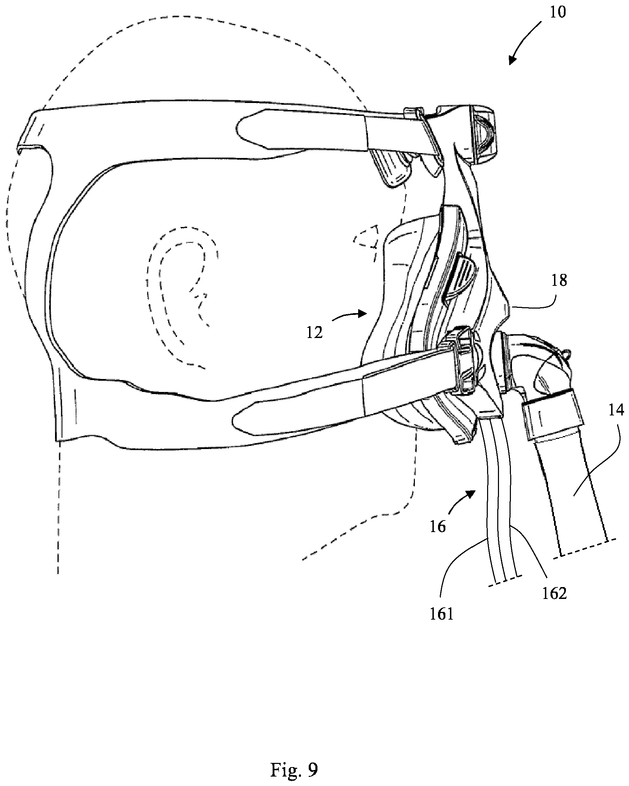

In forms of the present technology where liquid fluid is supplied towards the cushion two lines may be required as e.g. indicated by lines 161 and 162 in, e.g., Fig. 9. One line for supplying and one for removing fluid to achieve a circulation. This form of the mask including two lines (e.g. 161, 162) may be typically applicable for the case where the fluid is heated outside of the mask such as by or in the control unit. In forms of the present technology where heating and/or cooling elements may be implemented directly in the mask body, a single line for e.g. electrical communication between these heating/cooling elements and e.g. the control unit may be sufficient for masks implemented to operate with liquid and/or gaseous fluid(s).

Command line 16 may communicate various types of signals towards cushion 12 and/or receive various types of signals therefrom. Examples of such signals may include: electrical signals, hydraulic signals, optical signals (etc.) with these signals accordingly being aimed at altering or inciting change to one or more properties of the fluid in cushion 12. Typically, an electrical signal may be utilized where sensors are integral with the mask, a pressure signal communicated by e.g. a pressure line may be utilized if e.g. a pressure sensor is outside of the mask such as in the control unit, and no line may be required if, e.g., temperature sensor is in the control unit. Command line 16 may also be used to control or change the operation of the means in interface 10 that e.g. have been described with respect to Fig. 1 to operate in a pre-determined manner.

An electrical signal communicated via command line 16 towards cushion 12, shown for example in Fig. 8, may incite change to e.g. pressure, flow or temperature of fluid in cushion 12 by communicating the signal to a micro pump (e.g. a piezoelectric micro pump) that may be installed in the patient interface including also optionally in the cushion The micro pump is desirably a piezoelectric membrane pump, but any other pump able to handle both gaseous and liquid fluids in small increments with a required accuracy will do - e.g it could be a turbine pump as well. The micro pump may communicate with the cushion interior through the communication line, the pump may preferably be mounted outside to the mask body to avoid adding weight to the mask while being physically or operatively part of the control unit as may also be the heat sinks and heating elements.

According to the signal received, the micro pump may increase or decrease the pressure and/or flow of fluid in the cushion. This may be achieved by regulating the amount of fluid pumped into and/or the amount of fluid allowed to flow out of cushion 12 via a lumen provided e.g. also in command line 16. In this example, the communication of the electrical signal via command line 16 may be provided by a wire installed in a peripheral wall of command line 16 that surrounds the lumen through which command line 16 also provides the fluid communication with interface 10.

Fluid pumped into cushion 12 may be heated or cooled relative to the temperature of the fluid already in the cushion thereby preferably achieving the affect of inciting change to the temperature of fluid in cushion 12. Such heating of the fluid pumped into cushion 12 may be implemented by providing a heat exchanger (not shown) in a control unit (not shown) that communicates with interface 10 and by a separate hose(s) feeding such temperated liquid from the control unit to the cushion. Another exemplary way of inciting change to the temperature of fluid in the cushion by an electrical signal may be by communicating the electrical signal to e.g. a thermoelectric device adapted and arranged for changing the fluid temperature in the cushion that may be installed in the patient interface. Voltage changes incited to the thermoelectric device may preferably be either used to heat or cool the fluid in cushion 12. Device used for heating and cooling the fluid may be any device according to present industry standard and able to convert electrical energy into either heat, cold or both. Examples could be any or a combination of hot wire, heat sinks, cooling fans or Peltier elements. And, preferably these devices may be located integral with the control unit.

Attention is now drawn to Fig. 9. In accordance with some forms of the present technology, command line 16 may accordingly include first and second lines 161, 162 for communicating with cushion 12. First line 161 may be used for example for communicating fluid towards cushion 12 and second line 162 for communicating fluid out of and away from cushion 12. Fluid may be pumped towards or away from cushion 12 by for example a micro pump installed in interface 10 or an external pump (both not shown). A controller (not shown) controlling for example an optional valve (not shown) installed to second line 162 may be used to assist in regulating the fluid pressure in cushion 12 by e.g. closing or limiting an outgoing flow of fluid via line 162 while fluid is being pumped into the cushion via first line 161. A controlled valve may be needed in case the micro pump works "one way" only, so as to decrease the pressure in the system. If a pump is implemented that can communicate pressure both ways, then no extra valve may be required. Typically, all of the components may be located in the control unit.

The fluid flowing towards cushion 12 may be pressurized and/or at a temperature that is different from that of the fluid in cushion 12. For example, the fluid entering cushion 12 may be at a temperature that is cooler than the temperature of the fluid in cushion 12 to thereby cool the temperature of fluid in cushion 12.

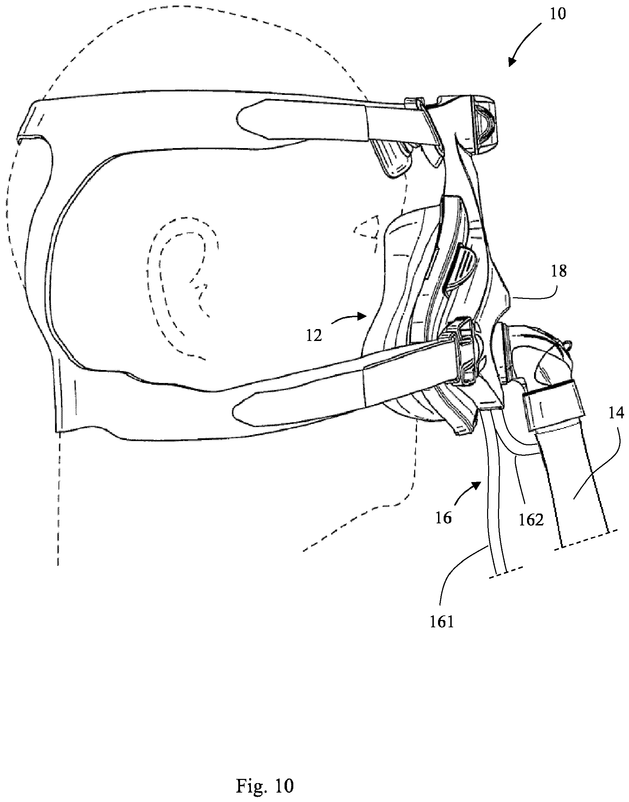

Attention is now drawn to Fig. 10. In accordance with some forms of the present technology, fluid flowing towards or away from cushion 12 may optionally flow at least part of the way along a fluid path (not shown) that is formed along the breathing hose 14.

This fluid path which is separated from the path for the breathing gases in breathing hose 14 may be provided for example along an outer periphery of breathing hose 14. In the example shown in Fig. 10, second line 162 of command line 16 is illustrated communicating between a distal portion of breathing hose 14 adjacent to where breathing hose 14 connects to patient interface 10, however other configurations may also be possible in accordance with the present technology. For example, the patient interface may be configured to include a fluid line (analogous to second line 162 in Fig. 10) for communicating between cushion 12 and e.g. a proximal end of breathing hose 14 where the fluid path in breathing hose 14 reaches interface 10.

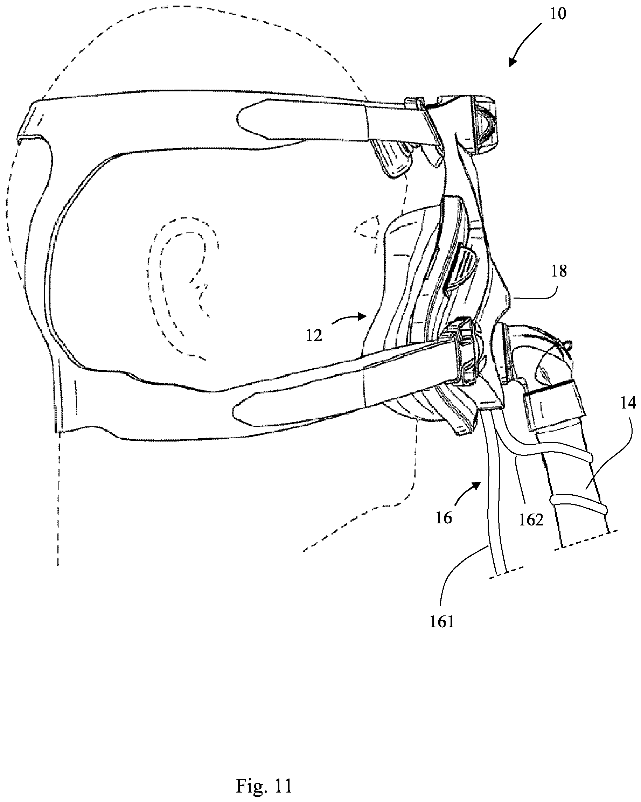

Heated fluid flowing towards or away from cushion 12 that passes through the fluid path in breathing hose 14 may optionally be used also for reducing condensation formed by breathing gaseous within the breathing hose. Optimal reduction of condensation at breathing hose 14 may be achieved if such heated fluid flows towards cushion 12 first via the fluid path in breathing hose 14 and then via second line 162, however also a reverse flow where the heated fluid flows first towards cushion 12 via first line 161 and then away from cushion 12 via second line 162 and via the fluid path in breathing cushion 14 may also be effective for reducing condensation, while increasing e.g. the heating effect obtained at cushion 12 Attention is now drawn to Fig. 11. In accordance with yet another form of the present technology, at least one of the lines 161, 162 (here only 162) leading fluid towards or away from cushion 12 may be configured to extend along breathing hose 14, here also wrap around breathing hose 14. Heated fluid flowing towards or away from cushion 12 via line 161 and/or 162 may be used to heat breathing hose 14 in order to assist in reducing condensation within breathing hose 14.

While the disclosure has been illustrated and described in detail in the drawings and foregoing description, such illustration and description are to be considered illustrative or exemplary and non-restrictive; the disclosure is thus not limited to the disclosed forms. Variations to the disclosed forms can be understood and effected by those skilled in the art and practicing the claimed disclosure, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures can not be used to advantage. Any reference signs in the claims should not be considered as limiting the scope.

## Claims

1. A patient interface (10, 110) for providing breathable gas to a patient, comprising a face engaging cushion (12), the cushion (12) comprising an internal cavity (13) that is adapted to contain fluid and a control means (120) being capable of communicating with the fluid for inciting change to at least a pressure of the fluid, **characterized in that** at last the pressure of the fluid can be actively changed in response to a pressure of the breathable gas;
and **in that**
• for a rise in the pressure of the breathable gas, the pressure of fluid in the cavity decreases, and/or for a decrease in the pressure of the breathable gas, the pressure of fluid in the cavity rises; or
• for a rise in the pressure of the breathable gas, the pressure of fluid in the cavity rises, and/or for a decrease in the pressure of the breathable gas, the pressure of fluid in the cavity decreases.

2. The patient interface (10, 110) according to claim 1, wherein decreasing the internal cavity pressure with increasing pressure of the breathable gas relaxes the cushion (12) during an inspiratory phase of the patient, and increasing the internal cavity pressure with decreasing pressure of the breathable gas stiffens the cushion (12) during an expiratory phase of the patient.

3. The patient interface (10, 110) according to claim 1, wherein increasing the internal cavity pressure with increasing pressure of the breathable gas stiffens the cushion (12) during an inspiratory phase of the patient, and decreasing the internal cavity pressure with decreasing pressure of the breathable gas relaxes the cushion (12) during an expiratory phase of the patient.

4. The patient interface (10, 110) according to any of the preceding claims, wherein pressure swings between the pressure of the breathable gas and the internal pressure of the cushion (12) may counteract each other.

5. The patient interface (10, 110) according to any of the preceding claims, wherein changes to a pressure of a gaseous fluid in the cushion (12) are effected by forming pressure swings in a fixed interval.

6. The patient interface (10, 110) according to any of the preceding claims, wherein the at least one property comprises temperature.

7. The patient interface (10, 110) according to claim 6, wherein the temperature is lowered or raised.

8. The patient interface (10, 110) according to any of the preceding claims, further comprising at least one second internal cavity (13) in the cushion (12) for containing fluid.

9. The patient interface (10, 110) according to claim 8, the patient interface (10, 110) being adapted to facilitate formation of difference between the cavities (13) in a given property.

10. The patient interface (10, 110) according to any one of the preceding claims, wherein active change is controlled automatically, optionally by an interface-means in or in communication with the control means (120), that preferably allows the patient or a physician to set upper and lower boundaries of the at least one property and/or a duration of time during which inciting of the at least one property will occur.

11. The patient interface (10, 110) according to any one of the preceding claims, wherein the cushion (12) comprises a membrane having a first side for contacting a patient's face during use and a second opposing side facing into a cushion cavity, and wherein the membrane is permeable to at least some fluid substances contained in the cavity (13), wherein the permeability is preferably a controlled permeability.

12. The patient interface (10, 110) according to claim 11, wherein the at least some fluid substances comprise skin care substances, optionally in the form of paste-like substances.

13. The patient interface (10, 110) according to any one of the preceding claims, wherein the fluid comprises at least one of: water, air, oil, silicone.

14. The patient interface (10, 110) according to any one of the preceding claims, wherein the interface comprises a pump, preferably a micro-pump, for at least partially urging communication with and/or increasing/decreasing flow and/or pressure of the fluid.

## Patentansprüche

1. Patientenschnittstelle (10, 110) zum Bereitstellen von Atemgas für einen Patienten mit einem Gesichtseingriffspolster (12), wobei das Polster (12) einen Innenhohlraum (13) aufweist, der geeignet ist, Fluid zu enthalten, und einer Steuereinrichtung (120), die mit dem Fluid zum Bewirken einer Änderung mindestens eines Drucks des Fluids kommunizieren kann, **dadurch gekennzeichnet, dass** mindestens der Druck des Fluids als Reaktion auf einen Druck des Atemgases aktiv geändert werden kann;
und dadurch, dass
- für einen Anstieg des Drucks des Atemgases der Fluiddruck im Hohlraum abnimmt und/oder für eine Abnahme des Drucks des Atemgases der Fluiddruck im Hohlraum ansteigt; oder
- für einen Anstieg des Drucks des Atemgases der Fluiddruck im Hohlraum ansteigt und/oder für eine Abnahme des Drucks des Atemgases der Fluiddruck im Hohlraum abnimmt.

2. Patientenschnittstelle (10, 110) nach Anspruch 1, wobei das Abnehmen des Innenhohlraumdrucks mit steigendem Druck des Atemgases das Polster (12) während einer Inspirationsphase des Patienten entspannt und das Ansteigen des Innenhohlraumdrucks mit abnehmendem Druck des Atemgases das Polster (12) während einer Exspirationsphase des Patienten versteift.

3. Patientenschnittstelle (10, 110) nach Anspruch 1, wobei das Ansteigen des Innenhohlraumdrucks mit steigendem Druck des Atemgases das Polster (12) während einer Inspirationsphase des Patienten versteift und das Abnehmen des Innenhohlraumdrucks mit abnehmendem Druck des Atemgases das Polster (12) während einer Exspirationsphase des Patienten entspannt.

4. Patientenschnittstelle (10, 110) nach einem der vorstehenden Ansprüche, wobei Druckwechsel zwischen dem Druck des Atemgases und dem Innendruck des Polsters (12) einander entgegenwirken können.

5. Patientenschnittstelle (10, 110) nach einem der vorstehenden Ansprüche, wobei Änderungen eines Drucks eines gasförmigen Fluids im Polster (12) durch Erzeugen von Druckwechseln in einem festen Intervall bewirkt werden.

6. Patientenschnittstelle (10, 110) nach einem der vorstehenden Ansprüche, wobei die mindestens eine Eigenschaft Temperatur aufweist.

7. Patientenschnittstelle (10, 110) nach Anspruch 6, wobei die Temperatur gesenkt oder erhöht wird.

8. Patientenschnittstelle (10, 110) nach einem der vorstehenden Ansprüche, ferner mit mindestens einem zweiten Innenhohlraum (13) im Polster (12) zum Aufnehmen von Fluid.

9. Patientenschnittstelle (10, 110) nach Anspruch 8, wobei die Patientenschnittstelle (10, 110) geeignet ist, die Erzeugung einer Differenz einer vorgegebenen Eigenschaft zwischen den Hohlräumen (13) zu erleichtern.

10. Patientenschnittstelle (10, 110) nach einem der vorstehenden Ansprüche, wobei das aktive Ändern automatisch gesteuert wird, optional durch eine Schnittstelleneinrichtung in der Steuereinrichtung (120) oder in Kommunikation mit ihr, die dem Patienten oder einem Arzt vorzugsweise ermöglicht, eine Ober- und Untergrenze der mindestens einen Eigenschaft und/oder eine Zeitdauer einzustellen, in deren Verlauf das Bewirken der mindestens einen Eigenschaft geschieht.

11. Patientenschnittstelle (10, 110) nach einem der vorstehenden Ansprüche, wobei das Polster (12) eine Membran aufweist, die eine erste Seite zum Kontaktieren des Gesichts eines Patienten im Gebrauch und eine zweite gegenüberliegende Seite hat, die in einen Polsterhohlraum weist, und wobei die Membran für mindestens einige Fluidsubstanzen durchlässig ist, die im Hohlraum (13) enthalten sind, wobei die Durchlässigkeit vorzugsweise eine gesteuerte Durchlässigkeit ist.

12. Patientenschnittstelle (10, 110) nach Anspruch 11, wobei die mindestens einigen Fluidsubstanzen Hautpflegesubstanzen aufweisen, optional in Form von pastösen Substanzen.

13. Patientenschnittstelle (10, 110) nach einem der vorstehenden Ansprüche, wobei das Fluid Wasser, Luft, Öl und/oder Silikon aufweist.

14. Patientenschnittstelle (10, 110) nach einem der vorstehenden Ansprüche, wobei die Schnittstelle eine Pumpe, vorzugsweise eine Mikropumpe, zum mindestens teilweisen Erzwingen von Kommunikation mit dem Fluid und/oder Erhöhen/Verringern von Durchfluss und/oder Druck des Fluids aufweist.

## Revendications

1. Interface patient (10, 110) pour administrer un gaz respirable à un patient, comprenant un coussin (12) venant en contact avec le visage, le coussin (12) comprenant une cavité interne (13) qui est adaptée pour contenir un fluide et un dispositif de commande (120) qui est en mesure de communiquer avec le fluide pour solliciter une modification par rapport à au moins une pression du fluide, **caractérisée en ce que** l'au moins une pression du fluide peut être activement modifiée en réponse à une pression du gaz respirable ;
et **en ce que**
• pour une augmentation de la pression du gaz respirable, la pression d'un fluide dans la cavité diminue, et/ou pour une diminution de la pression du gaz respirable, la pression d'un fluide dans la cavité augmente ; ou
• pour une augmentation de la pression du gaz respirable, la pression d'un fluide dans la cavité augmente, et/ou pour une diminution de la pression du gaz respirable, la pression d'un fluide dans la cavité diminue.

2. Interface patient (10, 110) selon la revendication 1, dans laquelle la diminution de la pression de cavité interne avec l'augmentation de la pression du gaz respirable détend le coussin (12) pendant une phase inspiratoire du patient, et l'augmentation de la pression de cavité interne avec la diminution de la pression du gaz respirable rigidifie le coussin (12) pendant une phase expiratoire du patient.

3. Interface patient (10, 110) selon la revendication 1, dans laquelle l'augmentation de la pression de cavité interne avec l'augmentation de la pression du gaz respirable rigidifie le coussin (12) pendant une phase inspiratoire du patient, et la diminution de la pression de cavité interne avec la diminution de la pression du gaz respirable détend le coussin (12) pendant une phase expiratoire du patient.

4. Interface patient (10, 110) selon l'une quelconque des revendications précédentes, dans laquelle les oscillations de pression entre la pression du gaz respirable et la pression interne du coussin (12) peuvent se contrecarrer.

5. Interface patient (10, 110) selon l'une quelconque des revendications précédentes, dans laquelle des modifications par rapport à une pression d'un fluide gazeux dans le coussin (12) sont effectuées par la création d'oscillations de pression dans un intervalle fixe.

6. Interface patient (10, 110) selon l'une quelconque des revendications précédentes, dans laquelle la au moins une propriété comprend la température.

7. Interface patient (10, 110) selon la revendication 6, dans laquelle la température est abaissée ou élevée.

8. Interface patient (10, 110) selon l'une quelconque des revendications précédentes, comprenant en outre au moins une deuxième cavité interne (13) dans le coussin (12) pour contenir un fluide.

9. Interface patient (10, 110) selon la revendication 8, l'interface patient (10, 110) étant adaptée pour faciliter la création d'une différence entre les cavités (13) dans une propriété donnée.

10. Interface patient (10, 110) selon l'une quelconque des revendications précédentes, dans laquelle une modification active est commandée automatiquement, optionnellement par un dispositif d'interface dans le dispositif de commande (120) ou en communication avec celui-ci, qui de préférence permet au patient ou à un médecin de déterminer des limites supérieure et inférieure de l'au moins une propriété et/ou une période de temps durant laquelle se produit la sollicitation de l'au moins une propriété.

11. Interface patient (10, 110) selon l'une quelconque des revendications précédentes, dans laquelle le coussin (12) comprend une membrane ayant un premier côté pour entrer en contact avec le visage d'un patient lors d'une utilisation et un deuxième côté opposé faisant face à une cavité de coussin, et dans laquelle la membrane est perméable à au moins certaines substances fluides contenues dans la cavité (13), dans laquelle la perméabilité est de préférence une perméabilité régulée.

12. Interface patient (10, 110) selon la revendication 11, dans laquelle les au moins certaines substances fluides comprennent des substances pour le soin de la peau, optionnellement sous la forme de substances de type pâte.

13. Interface patient (10, 110) selon l'une quelconque des revendications précédentes, dans laquelle le fluide comprend au moins l'un parmi : de l'eau, de l'air, de l'huile, du silicone.

14. Interface patient (10, 110) selon l'une quelconque des revendications précédentes, dans laquelle l'interface comprend une pompe, de préférence une micropompe, pour au moins partiellement provoquer une communication avec le fluide et/ou augmenter/diminuer un débit et/ou une pression du fluide.
